# FASCICULE DE BREVET EUROPEEN

(11) **EP 1 103 245 B1**
(45) Date de publication et mention de la délivrance du brevet: **14.06.2006**
(21) Numéro de dépôt: 00403005.2
(22) Date de dépôt: 27.10.2000
(51) Int. Cl.: A61K 8/81, A61Q 19/00

(54) **Composition cosmétique contenant un copolymère styrène/acrylique, et ses utilisations**
Kosmetische Zusammensetzung enthaltend ein Styrol/Acrylsäure Copolymer, und ihre Verwendungen
Cosmetic composition containing a styrene/acrylic copolymer, and its uses

(30) Priorité: 24.11.1999 FR 9914786
(43) Date de publication de la demande: 30.05.2001
(73) Titulaire: L'OREAL, 75008 Paris (FR)
(72) Inventeur: Chevalier, Véronique, 94400 Villecresnes (FR); Hurel, Valérie, 91190 Gif s/Yvette (FR)
(74) Mandataire: Boulard, Denis

(56) Documents cités:
- EP-A- 0 875 243
- BE-A- 725 083
- JP-A- 8 109 119
- US-A- 5 294 435
- PATENT ABSTRACTS OF JAPAN vol. 003, no. 075 (C-050), 27 juin 1979 (1979-06-27) & JP 54 049338 A (SHISEIDO CO LTD), 18 avril 1979 (1979-04-18)

## Description

La présente demande concerne se rapporte à une composition cosmétique matifiante contenant un copolymère styrène/acrylique non filmogène sous forme de particules en dispersion aqueuse et au moins une charge, et à son utilisation pour le soin et/ou le maquillage de la peau, en particulier pour estomper les défauts du relief de la peau tels que les microreliefs, les rides, les pores, tout en conférant à celle-ci un aspect naturel, ainsi qu'à son utilisation pour le traitement des peaux grasses. La demande se rapporte aussi à l'utilisation d'un copolymère styrène/acrytique non filmogène sous forme de particules en dispersion aqueuse, comme agent matifiant dans une composition cosmétique.

Par définition, un produit matifiant est un produit qui empêche la peau de briller et qui unifie le teint. Les compositions de soin de la peau ou de maquillage ayant des propriétés matifiantes sont généralement utilisées pour résoudre les problèmes de brillance occasionnés par un excès de sébum, et pour améliorer la tenue du maquillage à long terme, le maquillage ayant tendance à se dégrader visuellement au cours de la journée. Ces compositions donnent un aspect mat à la peau, résultant d'un pouvoir diffusant de la lumière à la surface de la peau. Elles peuvent aussi être utilisées pour estomper les défauts de la peau, tels que les microreliefs, les rides, les ridules, les pores ou les variations de couleur.

Les compositions classiques dites matifiantes contiennent généralement des poudres adsorbant le sébum et l'huile excédentaire de la composition, non adsorbée par la peau. Parmi les poudres matifiantes d'origine naturelle ou synthétique, on peut citer notamment les charges telles que le talc, l'amidon, le mica, la silice, les poudres de nylon, les poudres de polyéthylène, la poly-bêta-alanine, les poudres de poly(méth)acrylate de méthyle. Ce type de charges présentent l'inconvénient de donner à la peau un aspect poudreux, pas naturel, qui peut même accentuer les défauts de la peau. De plus, les compositions les contenant sont généralement desséchantes à long terme et s'étalent difficilement. Leur effet matifiant est peu durable dans le temps.

Le document EP-A-0502769 décrit des compositions matifiantes apportant une couche translucide et un aspect naturel à la peau maquillée. Il s'agit de dispersions de particules sphériques dans un liant gras dans un rapport en poids charges/liant très spécifique. Pour avoir un effet matifiant, il faut une forte proportion de poudres et, de ce fait, ces compositions peuvent être desséchantes. En outre, elles ont tendance à pelucher lors de l'étalement et à donner un effet blanchissant à la peau en raison d'une forte concentration en poudres.

Il subsiste donc le besoin d'une composition matifiante confortable lors de l'application et qui ne provoque aucune irritation ou dessèchement de la peau après application.

La demanderesse a maintenant trouvé une composition surmontant les inconvénients de l'art antérieur en évitant l'utilisation d'une grande quantité de poudres.

La demanderesse a découvert de manière surprenante que l'utilisation d'un copolymère styrène non filmogène sous forme de particules en dispersion aqueuse, acrylique dans une composition cosmétique contenant une charge, conférait à cette composition après application sur la peau, un aspect mat de façon prolongée dans le temps, tout en étant très confortable et non desséchante.

La présente invention a pour objet l'utilisation cosmétique d'une composition cosmétique contenant dans un milieu physiologiquement acceptable, au moins un copolymère styrène/acrylique non filmogène sous forme de particules en dispersion aqueuse et au moins une charge, pour donner un aspect mat à la peau.

On entend par milieu physiologiquement acceptable dans la composition de l'invention, un milieu non toxique et susceptible d'être appliqué sur la peau (y compris l'intérieur des paupières) ou les lèvres d'êtres humains.

On entend par « aspect mat » de la peau, un aspect non brillant, avec un teint unifié.

La composition de l'invention grâce à la présence du copolymère styrène/acrylique, est matifiante, c'est-à-dire qu'elle permet de donner un aspect mat à la peau. Aussi, l'invention a encore pour objet l'utilisation cosmétique d'un copolymère styrène/acrylique non filmogène sous forme de particules en dispersion aqueuse, comme agent matifiant dans une composition cosmétique.

Le copolymère styrène/acrylique se présente sous forme de particules en dispersion aqueuse, c'est-à-dire sous forme de particules en dispersion stable dans un milieu aqueux. Les particules de copolymère sont des particules sphériques ou pratiquement sphériques ayant de préférence une dimension moyenne en nombre allant de 0,1 à 5 µm.

On entend ici par milieu aqueux aussi bien un milieu contenant seulement de l'eau qu'un milieu comprenant de l'eau et un solvant hydrosoluble tel qu'un alcool inférieur comportant 1 à 6 atomes de carbone ou un glycol.

Le copolymère styrène/acrylique utilisé dans la composition de l'invention n'est pas un polymère filmogène, ce qui signifie qu'après évaporation de l'eau de la dispersion, ce copolymère ne donne pas un film, mais que les particules de copolymère contenant de l'air restent à la surface du support (en particulier de la peau) et diffusent la lumière dans toutes les directions. Un tel copolymère a une température de transition vitreuse (Tg) supérieure à 40°C, alors que les polymères filmogènes ont une température de transition vitreuse inférieure ou égale à 25°C.

Le copolymère styrène/acrylique est obtenu par polymérisation du styrène avec au moins un monomère choisi parmi l'acide acrylique, l'acide méthacrylique, un ester d'acide acrylique et un ester d'acide méthacrylique, le copolymère obtenu étant éventuellement réticulé. En outre, le copolymère peut être ou non neutralisé par une base inorganique telle que l'ammoniaque ou par une base organique.

Comme dispersions de copolymère styrène/acrylique non filmogène sous forme de particules en dispersion aqueuse, utilisables dans la composition de l'invention, on peut citer par exemple celles commercialisées sous la dénomination ROPAQUE OP-96 (dispersion aqueuse à 31 % de copolymère neutralisé par de l'ammoniaque), ROPAQUE OP-3000 (dispersion aqueuse à 37 % de copolymère) et ACUDYNE 290 (dispersion aqueuse à 40 % de copolymère) par la société Rohm & Haas.

La quantité de copolymère styrène/acrylique dans la composition de l'invention dépend de l'effet recherché. Le copolymère est généralement présent en une quantité en matière active allant de 0,1 à 20 %, de préférence de 0,5 à 10 % et mieux de 0,5 à 5 % en poids par rapport au poids total de la composition.

Comme charges pouvant être utilisées dans la composition de l'invention, on peut citer par exemple, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges. La quantité de charge(s) dans la composition de l'invention est de préférence inférieure à 15 % et va de préférence de 0,1 à 10 % en poids et mieux de 1 à 8 % en poids par rapport au poids total de la composition.

La composition peut se présenter sous forme d'une composition aqueuse ou sous forme d'une émulsion huile-dans-eau (H/E) ou eau-dans-huile (E/H) ou d'une émulsion multiple. Selon un mode préféré de l'invention, elle se présente sous forme d'émulsion notamment H/E ou E/H. On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique. Pour une utilisation pour les peaux grasses, on préfère avoir une émulsion H/E dont la phase aqueuse externe apporte un effet de fraîcheur.

La présente invention a encore pour objet une émulsion comprenant une phase aqueuse et une phase huileuse, caractérisée en ce qu'elle comprend au moins un copolymère styrène/acrylique non filmogène sous forme de particules en dispersion aqueuse et au moins une charge.

Quand la composition est une émulsion, elle comprend une phase huileuse. La nature de la phase huileuse de la composition de l'invention n'est pas critique et peut être constituée de tous les corps gras et notamment les huiles, classiquement utilisés dans le domaine cosmétique. La phase huileuse comprend au moins une huile.

Parmi les huiles utilisables dans la composition de l'invention, on peut notamment citer par exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl-benzoates), les huiles de silicone volatiles ou non volatiles telles que les polydiméthylsiloxanes (PDMS) et les cyclodiméthylsiloxanes ou cyclométhicones, notamment le cyclohexadiméthylsiloxane et le cyclopentadiméthylsiloxane, et les huiles fluorées ou fluorosiliconées. Les autres corps gras susceptibles d'être présents dans la phase huileuse peuvent être par exemple les acides gras, les alcools gras tels que l'alcool stéarylique, l'alcool cétylique et l'alcool cétéarylique, et les cires.

Dans les compositions de l'invention sous forme d'émulsion, la phase aqueuse de la composition peut être présente en une concentration allant de 1 à 80 % et de préférence 30 à 70 % en poids par rapport au poids total de la composition, et la phase huileuse peut être présente en une concentration allant de 5 à 70 % et de préférence de 10 à 50 % en poids par rapport au poids total de la composition.

Les émulsions peuvent contenir au moins un émulsionnant choisi parmi les émulsionnants amphotères, anioniques, cationiques ou non ioniques, utilisés seuls ou en mélange.

Les émulsionnants sont choisis de manière appropriée suivant l'émulsion à obtenir (E/H ou H/E).

Pour les émulsions H/E, on peut citer par exemple les émulsionnants suivants :
- comme émulsionnants amphotères, les N-acyl-aminoacides tels que les N-alkyl-aminoacétates et le cocoamphodiacetate disodique et les oxydes d'amines tels que l'oxyde de stéaramine ;
- comme émulsionnants anioniques, les acylglutamates tels que le "disodium hydrogenated tallow glutamate" (Amisoft HS-21^{R} commercialisé par la société AJINOMOTO) ; les acides carboxyliques et leurs sels tels que le stéarate de sodium ; les esters phosphoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ;
- comme émulsionnants cationiques, les alkyl-imidazolidinium tels que l'éthosulfate d'isostéaryl-éthylimidonium ; les sels d'ammonium tels que le chlorure de N,N,N-triméthyl-1-docosanaminium (Behentrimonium chloride) ;
- comme émulsionnants non ioniques, les esters et éthers d'oses tels que le stéarate de sucrose, le cocoate de sucrose, et le mélange de stéarate de sorbitan et de cocoate de sucrose commercialisé par la société ICI sous la dénomination d'Arlatone 2121^{R} ; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthylèné du mélange d'alcools gras en C12-C15 comportant 7 groupes oxyéthylénés (nom CTFA "C12-15 Pareth-7") ; les polymères d'éthylène glycol, tels que le PEG-100.

Pour les émulsions E/H, on peut citer par exemple comme émulsionnants les esters gras de polyol, notamment de glycérol ou de sorbitol, et notamment les esters isostéariques, oléiques et ricinoléiques de polyol, tels que le mélange de petrolatum, d'oléate de polyglycéryl-3, d'isostéarate de glycéryle, l'huile de ricin hydrogénée et d'ozokérite, vendu sous la dénomination Protegin W^{R} par la société GOLDSCHMIDT, l'isostéarate de sorbitan, le di-isostéarate de polyglycéryle, le sesqui-isostéarate de polyglycéryle-2 ; les esters et éthers d'oses tels que le "Methyl glucose dioleate" ; les esters gras tels que le lanolate de magnésium ; les dimethicone copolyols et alkyl-dimethicone copolyols tels que le Laurylmethicone copolyol vendu sous la dénomination Dow Corning 5200 Formulation Aid par la société DOW CORNING et le Cetyl dimethicone copolyol vendu sous la dénomination Abil EM 90^{R} par la société GOLDSCHMIDT.

Les émulsionnants peuvent être introduits tels quels ou sous forme de mélange avec d'autres émulsionnants et/ou avec d'autres composés tels que des alcools gras ou des huiles.

La composition de l'invention peut contenir en plus des adjuvants classiques tels que des colorants hydrosolubles ou liposolubles, des pigments, des parfums, des conservateurs, des filtres solaires, des séquestrants (EDTA), des actifs liposolubles ou hydrosolubles, des hydratants tels que les polyols et notamment la glycérine, des ajusteurs de pH (acides ou bases). Ces adjuvants peuvent être présents dans des quantités allant de préférence de 0,01 à 20 % en poids par rapport au poids total de la composition.

Comme actifs, on peut citer notamment les actifs utiles pour traiter les peaux grasses, tels que les sels de zinc et en particulier le gluconate de zinc ; les antibactériens comme l'acide salicylique, le triclosan, le lipacide, l'extrait de clou de girofle, l'octopirox, l'hexamidine ; les actifs anti-acné.

Par ailleurs, selon la fluidité de la composition que l'on souhaite obtenir, on peut y ajouter un ou plusieurs gélifiants hydrophiles ou lipophiles, choisis par exemple parmi les argiles, les gommes polysaccharides et leurs dérivés (gomme de xanthane, carboxyméthylcellulose, hydroxypropylguar), les polymères carboxyvinyliques ou carbomers, les polyacrylamides tels que celui commercialisé sous la dénomination SEPIGEL 305 par la société SEPPIC et les polymères d'acide acrylamidométhylpropanesulfonique au moins partiellement réticulés tels que le produit commercialisé sous la dénomination HOSTACERIN AMPS (nom CTFA : Ammonium polyacryldimethyltauramide) par la société HOECHST. Ces gélifiants sont généralement utilisés à des concentrations allant de 0,1 à 10 %, de préférence 0,1 à 5 % et mieux de 0,1 à 3 % du poids total de la composition.

La composition cosmétique selon l'invention trouve une application dans un grand nombre de traitements cosmétiques et/ou dermatologiques de la peau, et notamment en vue d'estomper les imperfections du relief de la peau, en particulier de camoufler les microreliefs, les rides et les ridules, les pores. Du fait de ses propriétés matifiantes, elle est également particulièrement appropriée pour le traitement des peaux grasses.

Aussi, un autre objet de l'invention consiste en l'utilisation cosmétique de la composition telle que définie précédemment, pour estomper les imperfections du relief de la peau et/ou pour camoufler les microreliefs, les rides et les ridules, les pores de la peau.

L'invention concerne également un procédé de traitement cosmétique de la peau destiné à lui apporter un aspect mat et/ou à camoufler les défauts du relief de la peau, caractérisé par le fait qu'on applique sur la peau une composition telle que définie précédemment.

L'invention a encore pour objet l'utilisation de la composition telle que définie précédemment pour la préparation d'une composition destinée au traitement des peaux grasses.

Les exemples qui suivent servent à illustrer l'invention sans toutefois présenter un caractère limitatif. Les quantités y sont données en % en poids, sauf mention contraire.

### Exemple 1 : Emulsion huile dans eau

### Phase huileuse :

- Alcool stéarylique 1 %
- Mélange de stéarate de glycéryle et de PEG-100
   (Simulsol 165 vendu par la société Seppic) 2 %
- Cyclohexadiméthylsiloxane 10 %

### Phase aqueuse :

- Glycérine 5 %
- Copolymère styrène/acrylique en dispersion aqueuse à 37 % (soit 2,96 % de matière active) 8 %
- Ammonium Polyacryldimethyltauramide
   (Hostacerin AMPS de la société Hoechst) 0,4 %
- Aluminium starch octenylsuccinate
   (DRY-FLO de la société National Starch) 3 %
- Gomme de xanthane 0,2 %
- Hydroxyde de sodium 0,01 %
- Conservateurs 0,7 %
- Eau qsp 100 %

Mode opératoire : l'émulsion est préparée en ajoutant, sous agitation, la phase huileuse chauffée à 65°C à la phase aqueuse chaude.

On obtient une composition apte à matifier la peau en supprimant la brillance initiale.

### Exemple 2 : Emulsion huile dans eau

### Phase huileuse :

- Alcool stéarylique 1 %
- Mélange dimyristyl tartrate/ceterayl alcohol/C12-C15-Pareth-7/PPG-25 laureth-25
   (Cosmacol PSE vendu par la société Enichem) 1,5 %
- Cyclohexadiméthylsiloxane 10 %

### Phase aqueuse :

- Glycérine 5 %
- Copolymère styrène/acrylique en dispersion aqueuse à 31 %, neutralisé par de la triéthanolamine
   (soit 0,91 % de matière active) 3 %
- Ammonium Polyacryldimethyltauramide
   (Hostacerin AMPS de la société Hoechst) 0,4 %
- Aluminium starch octenylsuccinate
   (DRY-FLO de la société National Starch) 3 %
- Gomme de xanthane 0,2 %
- Hydroxyde de sodium 0,01 %
- Conservateurs 0,7 %
- Eau qsp 100 %

Le mode opératoire est le même que celui de l'exemple 1.

On obtient une composition matifiante qui élimine la brillance de la peau.

### Exemple 3 : Emulsion huile dans eau

### Phase huileuse :

- Alcool stéarylique 1 %
- Mélange de stéarate de glycéryle et de PEG-100
   (Simulsol 165 vendu par la société Seppic) 2 %
- Cyclohexadiméthylsiloxane 10 %

### Phase aqueuse :

- Glycérine 5 %
- Copolymère styrène acrylique en dispersion aqueuse à 37 % (soit 1,11 % de matière active) 3 %
- Ammonium Polyacryldimethyltauramide
   (Hostacerin AMPS de la société Hoechst) 0,4 %
- Aluminium starch octenylsuccinate
   (DRY-FLO de la société National Starch) 3 %
- Gomme de xanthane 0,2 %
- Hydroxyde de sodium 0,01 %
- Conservateurs 0,7 %
- Eau qsp 100 %

Le mode opératoire est le même que celui de l'exemple 1.

On obtient une composition matifiante qui élimine la brillance de la peau.

### Exemple comparatif : Emulsion huile dans eau

### Phase huileuse :

- Alcool stéarylique 1 %
- Mélange de stéarate de glycéryle et de PEG-100
   (Simulsol 165 vendu par la société Seppic) 2 %
- Cyclohexadiméthylsiloxane 10 %

### Phase aqueuse :

- Glycérine 5 %
- Ammonium Polyacryldimethyltauramide
   (Hostacerin AMPS de la société Hoechst) 0,4 %
- Aluminium starch octenylsuccinate
   (DRY-FLO de la société National Starch) 3 %
- Silice 3 %
- Gomme de xanthane 0,2 %
- Hydroxyde de sodium 0,01 %
- Conservateurs 0,7 %
- Eau qsp 100 %

Test de matité : on a mesuré la matité obtenue pour les compositions de l'exemple 3 selon l'invention, comprenant 1,11 % de copolymère (en matière active), et de l'exemple comparatif comprenant 3 % de silice. La mesure a été réalisée de la manière suivante : sur un support en caoutchouc, on a étalé la composition à raison de 2 g/cm², on a laissé sécher, puis on a mesuré la réflexion à l'aide d'un gonioréflectomètre, le résultat obtenu étant le rapport R entre la réflexion spéculaire et la réflexion diffuse. La valeur de R est d'autant plus faible que l'effet matifiant est important.

| Composition | Exemple 3 | Exemple comparatif |
|---|---|---|
| R | 1,51 ± 0,075 | 2,29 ± 0,075 |

Ces résultats montrent qu'avec une concentration en copopolymère moins importante, on obtient un résultat de matiité bien supérieur à celui obtenu avec 3 % de silice.

## Revendications

1. Utilisation cosmétique d'une composition cosmétique contenant dans un milieu physiologiquement acceptable, au moins un copolymère styrène/acrylique non filmogène sous forme de particules en dispersion aqueuse et au moins une charge, pour donner un aspect mat à la peau.

2. Utilisation cosmétique d'une composition cosmétique contenant dans un milieu physiologiquement acceptable, au moins un copolymère styrène/acrylique non filmogène sous forme de particules en dispersion aqueuse et au moins une charge, pour estomper les imperfections du relief de la peau et/ou pour camoufler les microreliefs, les rides et les ridules, les pores de la peau.

3. Utilisation selon la revendication précédente, **caractérisée par le fait que** les particules de copolymère ont une dimension moyenne en nombre allant de 0,1 à 5 µm.

4. Utilisation selon l'une quelconque des revendications précédentes, **caractérisée par le fait que** le copolymère styrène/acrylique est obtenu par polymérisation du styrène avec au moins un monomère choisi parmi l'acide acrylique, l'acide méthacrylique, un ester d'acide acrylique et un ester d'acide méthacrylique.

5. Utilisation selon l'une quelconque des revendications précédentes, **caractérisée par le fait que** le copolymère est présent en une quantité en matière active allant de 0,1 à 20 % en poids par rapport au poids total de la composition.

6. Utilisation selon l'une quelconque des revendications précédentes, **caractérisée par le fait que** la charge est choisie parmi la poudre de silice ; le talc ; les particules de polyamide ; les poudres de polyéthylène ; les microsphères à base de copolymères acryliques ; les poudres expansées ; les poudres de matériaux organiques naturels ; les microbilles de résine de silicone ; et leurs mélanges.

7. Utilisation selon l'une quelconque des revendications précédentes, **caractérisée par le fait que** la composition contient une quantité de charge(s) allant de 0,1 à 10 % en poids par rapport au poids total de la composition.

8. Utilisation selon l'une quelconque des revendications précédentes, **caractérisée par le fait que** la composition se présente sous forme d'une émulsion.

9. Utilisation selon l'une quelconque des revendications précédentes, **caractérisée par le fait que** la composition contient en outre un ou plusieurs gélifiants.

10. Procédé de traitement cosmétique de la peau, destiné à lui apporter un aspect mat et/ou à camoufler les défauts du relief de la peau, **caractérisé par le fait qu'**on applique sur la peau une composition contenant dans un milieu physiologiquement acceptable, au moins un copolymère styrène/acrylique non filmogène sous forme de particules en dispersion aqueuse et au moins une charge.

11. Utilisation cosmétique d'un copolymère styrène/acrylique non filmogène sous forme de particules en dispersion aqueuse, comme agent matifiant dans une composition cosmétique.

12. Emulsion comprenant une phase aqueuse et une phase huileuse, **caractérisée en ce qu'**elle comprend au moins un copolymère styrène/acrylique non filmogène sous forme de particules en dispersion aqueuse et au moins une charge.

13. Emulsion selon la revendication 12, **caractérisée en ce que** les particules du copolymère ont une dimension moyenne en nombre allant de 0,1 à 5 µm.

## Claims

1. Cosmetic use of a cosmetic composition containing, in a physiologically acceptable medium, at least one non film-forming styrene/acrylic copolymer in the form of particles in aqueous dispersion and at least one filler, for giving a matt appearance to the skin.

2. Cosmetic use of a cosmetic composition containing, in a physiologically acceptable medium, at least one non film-forming styrene/acrylic copolymer in the form of particles in aqueous dispersion and at least one filler, for fading out skin relief imperfections and/or for camouflaging skin microreliefs, wrinkles, fine lines and pores.

3. Use according to the preceding claim, **characterized in that** the copolymer particles have an average numerical, size ranging from 0.1 µm to 5 µm.

4. Use according to any one of the preceding claims, **characterized in that** the styrene/acrylic copolymer is obtained by polymerizing styrene with at least one monomer chosen from acrylic acid, methacrylic acid, an acrylic acid ester and a methacrylic acid ester.

5. Use according to any one of the preceding claims, **characterized in that** the copolymer is present in an amount of active material ranging from 0.1% to 20% by weight relative to the total weight of the composition.

6. Use according to any one of the preceding claims, **characterized in that** the filler is chosen from silica powder; talc; polyamide particles; polyethylene powders; microspheres made of acrylic copolymers; expanded powders; powders of natural organic materials; silicone resin microbeads; and mixtures thereof.

7. Use according to any one of the preceding claims, **characterized in that** the composition contains an amount of filler (s) ranging from 0.1% to 10% by weight relative to the total weight of the composition.

8. Use according to any one of the preceding claims, **characterized in that** the composition is in the form of an emulsion.

9. Use according to any one of the preceding claims, **characterized in that** the composition also contains one or more gelling agents.

10. Cosmetic treatment process for the skin which is intended to give it a matt appearance and/or to camouflage skin relief defects, **characterized in that** a composition containing, in a physiologically acceptable medium, at least one non film-forming styrene/acrylic copolymer in the form of particles in aqueous dispersion and at least one filler is applied to the skin.

11. Cosmetic use of a non film-forming styrene/acrylic copolymer in the form of particles in aqueous dispersion, as a matt-effect agent in a cosmetic composition.

12. Emulsion comprising an aqueous phase and an oily phase, **characterized in that** it comprises at least one non film-forming styrene/acrylic copolymer in the form of particles in aqueous dispersion and at least one filler.

13. Emulsion according to Claim 12, **characterized in that** the particles of the copolymer have an average numerical size ranging from 0.1 µm to 5 µm.

## Patentansprüche

1. Kosmetische Verwendung einer kosmetischen Zusammensetzung, die in einem physiologisch akzeptablen Medium mindestens ein nichtfilmbildendes Styrol/Acryl-Copolymer in Form von Partikeln in wässeriger Dispersion und mindestens einen Füllstoff enthält, um der Haut ein mattes Aussehen zu geben.

2. Kosmetische Verwendung einer kosmetischen Zusammensetzung, die in einem physiologisch akzeptablen Medium mindestens ein nichtfilmbildendes Styrol/Acryl-Copolymer in Form von Partikeln in wässeriger Dispersion und mindestens einen Füllstoff enthält, um die Unzulänglichkeiten des Hautreliefs zu verdecken und/oder Mikroreliefe, Falten und Fältchen und Poren der Haut abzudecken.

3. Verwendung nach dem vorhergehenden Anspruch, **dadurch gekennzeichnet, dass** die Copolymerpartikel eine zahlenmittlere Abmessung von 0,1 bis 5 µm aufweisen.

4. Verwendung nach dem vorhergehenden Anspruch, **dadurch gekennzeichnet, dass** das Styrol/Acryl-Copolymer durch Polymerisation von Styrol mit mindestens einem Monomer hergestellt wird, das unter Acrylsäure, Methacrylsäure, Acrylsäureestern und Methacrylsäureestern ausgewählt ist.

5. Verwendung nach dem vorhergehenden Anspruch, **dadurch gekennzeichnet, dass** das Copolymer in einer Wirkstoffmenge von 0,1 bis 20 Gew.-%, bezogen auf das Gesamtgewicht der Zusammensetzung, enthalten ist.

6. Verwendung nach dem vorhergehenden Anspruch, **dadurch gekennzeichnet, dass** der Füllstoff unter Siliciumdioxidpulver; Talk, Polyamidpartikeln; Polyethylenpartikeln; Mikrosphären auf der Basis von Acrylcopolymeren; expandierten Pulvern; Pulvern aus natürlichen organischen Stoffen; Siliconharzmikrokugeln und deren Gemischen ausgewählt ist.

7. Verwendung nach dem vorhergehenden Anspruch, **dadurch gekennzeichnet, dass** die Zusammensetzung einen Mengenanteil des Füllstoffes oder der Füllstoffe von 0,1 bis 10 Gew.-%, bezogen auf das Gesamtgewicht der Zusammensetzung, enthält.

8. Verwendung nach dem vorhergehenden Anspruch, **dadurch gekennzeichnet, dass** die Zusammensetzung als Emulsion vorliegt.

9. Verwendung nach dem vorhergehenden Anspruch, **dadurch gekennzeichnet, dass** die Zusammensetzung ferner einen oder mehrere Gelbildner enthält.

10. Verfahren zur kosmetischen Behandlung der Haut, das dazu dient, die Haut matt aussehen zu lassen und/oder Unzulänglichkeiten des Hautreliefs zu verbergen, **dadurch gekennzeichnet, dass** auf die Haut eine Zusammensetzung aufgetragen wird, die in einem kosmetisch akzeptablen Medium mindestens ein nichtfilmbildendes Styrol/Acryl-Copolymer in Form von Partikeln in wässeriger Dispersion und mindestens einen Füllstoff enthält.

11. Kosmetische Verwendung eines nichtfilmbildenden Styrol/Acryl-Copolymers in Form von Partikeln in wässeriger Dispersion als Mattierungsmittel in einer kosmetischen Zusammensetzung.

12. Emulsion, die eine wässerige Phase und eine Ölphase enthält, **dadurch gekennzeichnet, dass** sie mindestens ein nichtfilmbildendes Styrol/Acryl-Copolymer in Form von Partikeln in wässeriger Dispersion und mindestens einen Füllstoff enthält.

13. Emulsion nach Anspruch 12, **dadurch gekennzeichnet, dass** die Partikel des Copolymers eine zahlenmittlere Abmessung von 0,1 bis 5 µm aufweisen.
